# EUROPEAN PATENT APPLICATION

(11) **EP 1 964 934 A1**
(43) Date of publication of application: **03.09.2008**
(21) Application number: 08152040.5
(22) Date of filing: 28.02.2008
(51) Int. Cl.: C12Q 1/68

(54) **Sample treatment solution and reagent kit for preparing sample for detecting DNA methylation**

(30) Priority: 28.02.2007 JP 2007050748
(71) Applicant: SYSMEX CORPORATION, Kobe-shi, Hyogo 651-0073 (JP)
(72) Inventor: Masahiro, Kajita c/o Sysmex Corporation, Kobe-shi Hyogo 651-0073 (JP); Ayako, Sakai c/o Sysmex Corporation, Kobe-shi Hyogo 651-0073 (JP); Noriaki, Yamamoto c/o Sysmex Corporation, Kobe-shi Hyogo 651-0073 (JP); Hideki, Ishihara c/o Sysmex Corporation, Kobe-shi Hyogo 651-0073 (JP)
(74) Representative: De Clercq, Ann G. Y.

(57) **Abstract**

The present invention provides a sample treatment solution for preparing a sample for DNA methylation which can achieve stable detection results in detection of DNA methylation and be easily pretreated, comprising an aqueous solution containing a protease.

## Description

### FIELD OF THE INVENTION

The present invention relates to a sample treatment solution and a reagent kit, which are used in preparing a sample for detecting DNA methylation. The present invention also relates to a method of preparing a sample, which comprises using the sample treatment solution and the reagent kit, as well as a method of detecting DNA methylation.

### BACKGROUND

With reference to a chromosome DNA in higher eukaryotes, the 5th position of C (cytosine) among bases constituting DNA may be modified by methylation. Unlike in the case of prokaryotes, the DNAmethylation in higher eukaryotes functions as a mechanism for inhibiting expression of genetic information. For example, when a region containing a large number of CpG of a certain gene (CpG island) is methylated, transcription of the gene is inhibited. By contrast, when the CpG island is not methylated, a transcription factor can be bound to a promoter region and gene transcription can proceed. Thus, DNA methylation is one of control mechanisms of gene expression and plays an important role in various physiological and pathological phenomena such as early embryonic development, tissue-specific gene expression, genomic imprinting and X chromosome inactivation which are phenomena unique to mammals, chromosome stabilization, and timing of DNA replication. Furthermore, it has been revealed that the DNA methylation is strongly involved in cancer and other diseases in recent years.

In general, methylation-specific PCR (Methylation specific Polymerase Chain Reaction: hereinafter referred to as "MS-PCR") is used as a method of analyzing DNA methylation (James G. HERMAN et al., Methylation-specific PCR: A novel PCR assay for methylation status of CpG islands, Proc. Natl. Acad. Sci. USA, Vol. 93, pp.9821-9826, September 1996). A conventionally used method (hereinafter referred to as "standard method") is usually performed by the four steps: 1) Genomic DNA extraction from a biological sample (about 3 hours); 2) bisulfite treatment of the extracted genomic DNA (about 16 hours); 3) purification of the modified genomic DNA (about 1 hour); and 4) PCR (about 2 hours), which are very time-consuming (a total of about 22 hours). In Particular, pretreatment of the biological sample (preparation of the sample for detection) prior to PCR is complicated and it takes about 20 hours just to pretreat (the above steps 1 to 3).

In order to shorten such a pretreatment time, a method in which the bisulfite treatment in the above step 2) is performed with a high concentration of bisulfite has been suggested (Masahiko SHIRAISHI and Hikoya HAYATSU, High-Speed Conversion of Cytosine to Uracil in Bisulfite Genomic Sequencing Analysis of DNA Methylation, DNARESEARCH 11, 409-415 (2004)). According to this method (hereinafter referred to as "rapid method"), when the rapid method is used for bisulfite treatment which usually takes about 16 hours to complete with the standard method it can be completed in about 20 minutes. Therefore, the pretreatment time is reduced from about 20 hours to about 4 hours. However, even if the rapid method is used, it takes about 4 hours to pretreatment.

JP-A 2005-058217 discloses a method of detecting a methylated DNA, which comprises dissolving a very small amount of a cell sample with a solution containing a protein denaturant such as guanidine thiocyanate, sodium iodide, urea and SDS, and treating the resulting solution by the bisulfite treatment in the standard method described above.

### SUMMARY

The scope of the present invention is defined solely by the appended claims, and is not affected to any degree by the statements within this summary.

The present inventors found that the method described in JP-A 2005-058217 has the following problems:
1) the detection results of DNA methylation are not stable when the amount of a protein contained in a biological sample is high, and
2) the detection results are not stable because a protein in a sample subjected to bisulfite treatment is salted out in the rapid method of using a high concentration of bisulfite.

Accordingly, the object of the present invention is to provide a sample treatment solution and a reagent kit, which are used for preparing a sample for DNA methylation that can achieve stable detection results in detection of DNAmethylation and can be easily pretreated, as well as a method of preparing a sample and a method of detecting DNA methylation by using the sample treatment solution and the reagent kit.

The present inventors found that when a sample treatment solution comprising an aqueous solution containing a protease is used for a treatment of a biological sample, stable detection results of DNA methylation can be obtained, and the present invention was thereby completed.

According to the present invention, there can be provided a sample treatment solution and a reagent kit, which are used for preparing a sample for DNAmethylation that can achieve stable detection results in detection of DNA methylation and can be easily pretreated, as well as a method of preparing a sample and a method of detecting DNA methylation by using the sample treatment solution and the reagent kit.

The sample treatment solution, the reagent kit, the method of preparing a sample and the method of detecting DNAmethylation according to the present invention are useful for detecting DNA methylation by using a biological sample containing a large amount of protein. Particularly, the sample treatment solution, the reagent kit, the method of preparing a sample and the method of detecting DNA methylation according to the present invention are useful for detecting DNA methylation by using the rapid method.

The method of preparing a sample for detecting DNA methylation according to the present invention can simplify the extraction of genomic DNA. Accordingly, a sample for detection of DNA methylation can be prepared in a short time. In the method of preparing a sample in this embodiment, furthermore, pretreatment can be carried out in one container. Therefore, pipetting is not necessary in pretreatment. Accordingly, a loss in a sample caused by pipetting can be reduced. As a result, detection sensitivity can be improved.

Further, the method of preparing a sample for detecting DNAmethylation according to the present invention can be carried out easily in a few steps. Accordingly, this method is suitable for automation with an apparatus.

### BRIEF DESCRIPTION OF THE DRAWINGS

Fig. 1 is a schematic view of a reagent kit for preparing a sample for detection of DNA methylation.
Fig. 2 is a photograph showing results of methylation of a CpG island of ERα gene and E-cad gene with a sample treatment solution A, a sample treatment solution B and a control.
Fig. 3 is a photograph showing results of methylation of a CpG island of ERα gene and E-cad gene with sample treatment solutions A to F and a control.

### DETAILED DESCRIPTION OF THE EMBODIMENT

The method of preparing a sample for detecting DNA methylation in this embodiment comprises mixing a sample treatment solution comprising an aqueous solution containing a protease with a biological sample containing cells (mixing step) and adding a converting agent to the resulting mixture to convert non-methylated cytosine contained in a DNA in the mixture into another base (converting step).

In this embodiment, the aqueous solution is not particularly limited insofar as it contains water as a solvent, and the aqueous solution may contain an organic solvent such as alcohol.

The protease in this embodiment is not particularly limited as long as it is a proteolytic enzyme. Examples include thermolysin, thermoase, papain, trypsin, pronase, α-chymotrypsin, subtilisin, pepsin, plasmin and proteinase K. Particularly, proteinase K is preferable.

The concentration of a proteinase contained in a sample treatment solution may be suitably selected depending on the protease used and is not particularly limited. For example, when proteinase K is used, the concentration thereof in a sample treatment solution is preferably 0.5 to 40 mg/ml.

The biological sample in this embodiment is not particularly limited as long as it is a sample containing cells collected from the living body. Examples include tissues such as lymph nodes and blood, plasma, serum, urine, saliva, a body fluid and a bodily secretion of mammary gland, collected from animals such as humans, tissues collected from animals such as humans, culturedtissuesand cultured cells. Particularly, the biological sample is preferably serum, plasma, a biomedical tissue and a bodily secretion of mammary gland.

The sample treatment solution in this embodiment is used for preparing a sample for detection of DNA methylation from a biological sample containing cells. The sample treatment solution comprises an aqueous solution containing a protease. Particularly, a sample treatment solution comprising an aqueous solution further containing a surfactant and/or a chaotropic agent is preferable.

Any surfactants such as an anionic surfactant, a cationic surfactant, an amphoteric surfactant, and a nonionic surfactant maybe used as long as the above-mentioned cells can be solubili zed by the surfactants. Among them, an anionic surfactant is particularly preferable.

Preferable examples of such an anionic surfactant include sodium dodecyl sulfate (SDS), sodium tetradecyl sulfate, sodium dodecyl sulfonate, sodium tetradecyl sulfonate, sodium cholate (CHO), sodium deoxycholate (DOC), sodium taurocholate, sodium taurodeoxycholate, and the like. Among them, sodium dodecyl sulfate (SDS) which has a high affinity for protein is particularly preferable.

It is desirable that the concentration of a surfactant contained in a sample treatment solution is suitably selected depending on the type of a surfactant to be used. For example, the concentration of an anionic surfactant contained in a sample treatment solution is preferably 0.1 to 10 volume %, more preferably 0.5 to 5 volume %.

The chaotropic agent is not particularly limited as long as it has a property of destabilizing a molecular structure of water and acts on a hydrophobic molecule to weaken its hydrophobic bond thereby increasing the water solubility thereof. Examples of chaotropic agents include guanidine thiocyanate, sodium thiocyanate, guanidine hydrochloride, sodium iodide, potassium iodide and urea. Particularly, guanidine hydrochloride and urea are preferable.

It is desirable that the concentration of a chaotropic agent contained in a sample treatment solution is suitably selected depending on the type of a chaotropic agent to be used. For example, the concentration of guanidine hydrochloride contained in a sample treatment solution is preferably 0.5 to 8 M, more preferably 1 to 6 M. In addition, the concentration of urea contained in a sample treatment solution is preferably 1 to 24 M.

A pH of a sample treatment solution may be a pH range in which the surfactant can be dissolved. However, the pH is preferably from neutral to weak alkaline, particularly from pH 7 to 10 in order to promote decomposition of RNA in cells when the sample treatment solution is mixed with a biological sample. In order to keep the pH constant, the sample treatment solution may also contain known buffer solutions.

A pH of a sample treatment solution may be a pH range in which a protease can have an enzyme reaction. However, the pH is preferably from neutral to weak alkaline, particularly from pH 7 to 10 in order to promote decomposition of RNA in cells when the sample treatment solution is mixed with a biological sample. In order to keep the pH constant, the sample treatment solution may also contain known buffer solutions.

The mixing step in this embodiment is not particularly limited as long as a biological sample can be sufficiently mixed with a sample treatment solution, and a protein in the biological sample can react with a protease in the sample treatment solution. For example, the mixing step can be carried out by stirring a biological sample and a sample treatment solution at 25 to 70°C for 5 to 120 minutes.

An additive amount of the sample treatment solution is not particularly limited and may be suitably regulated depending on, for example, the amount of the biological sample and the concentration of a protease in the sample treatment solution. For example, 100 to 1000 µl, preferably 200 to 600 µl is desirable from the viewpoint of operability in the subsequent converting step.

A dissolving step of dissolving a biological sample containing cells with a dissolving solution containing a surfactant and/or a chaotropic agent can also be performed before the mixing step. After the cells contained in a biological sample are thus dissolved, the mixing step can be carried out thereby suitably decomposing a protein with a protease.

Conditions in the dissolving step are not particularly limited as long as cells can be dissolved with a surfactant and/or a chaotropic agent. For example, the dissolving step can be carried out by stirring at 25 to 70°C for 5 to 120 minutes.

An additive amount of the dissolving solution is not particularly limited and can be suitably regulated depending on, for example, the amount of a biological sample and the concentration of a surfactant and/or a chaotropic agent in the dissolving solution. For example, 100 to 1000 µl, preferably 200 to 600 µl, is desirable from the viewpoint of operability in the subsequent converting step.

Before the converting step is carried out, a step of physically disrupting cells contained in a biological sample may be conducted. For example, when cell mass such as an excised tissue is used as the biological sample, the cell mass is mixed with a sample treatment solution or a dissolving solution and then the cell mass is subjected to homogenization or the like.

The converting step in this embodiment is not particularly limited as long as non-methylated cytosine contained in a DNA can be converted into another base by adding a converting agent to the mixture obtained by the mixing step described above. From the viewpoint of reducing the reaction time, the rapid method described above is particularly preferable.

The converting agent converts unmethylated cytosine in DNA into different bases. Namely, it converts unmethylated cytosine into bases other than cytosine (uracil, thymine, adenine, or guanine). Bisulfite is preferably used as such converting agent. Sodium salt, potassium salt, calcium salt, magnesium salt, and the like can be used as bisulfite. When DNA is treated using any one of these converting agents, unmethylated cytosine in DNA are converted into uracil by deamination. On the other hand, the converting agents as described above have no effect on methylation cytosine in DNA, and therefore the conversion does not occur. Preferably, this converting agent is dissolved in a solvent and used as a solution.

When bisulfite is used as a converting agent, an additive amount is not particularly limited as long as the level is sufficient to convert unmethylated cytosine of DNA in a sample. However, it is 1 M and more, preferably 1 to 15 M, and more preferably 3 to 10 M. For example, in the case where 4 M of sodium bisulfite is added to the mixed solution, unmethylated cytosine can be converted into uracils by incubating them at 50 to 80°C for 10 to 90 minutes. As in the standard method described above, when bisulfite is used at low concentrations the conversion may be performed according to time and temperature which are sufficient to convert unmethylated cytosine. The converting agent is not limited to bisulfite. A converting agent which selectively converts unmethylated cytosine rather than methylation cytosine into different bases can also be used.

In the method of preparing the sample for detection, it is preferable to include the step of cleaving DNA prior to the second step.

In this embodiment, a step for cutting DNA may be included before the converting step. DNA contained in a biological sample is cleaved into the proper length to fragment the DNA, thereby converting non-methylated cytosine efficiently in the second step. The cleavage of DNA can be performed with a physical treatment such as sonication, a chemical treatment such as an alkaline treatment, an enzyme treatment with restriction enzymes, and the like. Among them, substances which can cleave DNA (cleavage agent) such as sodium hydroxide and restriction enzyme are preferably used. For example, when sodium hydroxide is used as a cleavage agent, DNA can be cleaved by adding 5 to 30 µl of 5 to 15N sodium hydroxide solution to the mixed solution obtained by the first step and then incubating them at 10 to 40°C for 5 to 15 minutes.

When DNA is cleaved with the chemical treatment and the enzyme treatment, a substance used for the alkaline treatment (for example, sodium hydroxide) or an enzyme (for example, restriction endonuclease) can be added to the above-mentioned sample treatment solution as cleavage agents. In this case, solubilization and cleavage of a sample can be performed in a single procedure simply by mixing a biological sample and a sample treatment solution, and therefore the time required for preparation of the sample for detection (pretreatment time). In the case where sodium hydroxide is used the concentration of sodium hydroxide in a sample treatment solution is preferably 0.1 to 1N, more preferably 0.2 to 0.5 N. DNA may be cleaved at any time prior to the addition of the converting agent.

Before the converting step is carried out, a step of converting a double-stranded DNA contained in a sample into a single-stranded DNA may also be carried out in this embodiment. By converting a double-stranded DNA into a single-stranded DNA, the conversion of non-methylated cytosine can be efficiently carried out in the converting step. In methylation specific PCR described later, a DNA polymerase easily binds to a DNA so that detection of DNA methylation can be facilitated.

The method of converting a double-stranded DNA into a single-stranded DNA includes, for example, a method of using an enzyme such as DNA helicase, a physical method of increasing the temperature of a DNA-containing solution, and a chemical method of alkali-treating a DNA with sodium hydroxide. Particularly, the chemical method of alkali-treating a DNA with sodium hydroxide is preferable because DNA cleavage can also be simultaneously conducted.

In this embodiment, a reagent kit used in preparing a sample for detecting DNAmethylation contains a first reagent comprising the above-mentioned sample treatment solution and a second reagent containing the converting agent.

The reagent kit in this embodiment can further contain the above dissolving solution and a cleaving agent. In this case, the sample treatment solution, the converting agent, the dissolving solution and the cleaving agent may be accommodated respectively in different containers. These materials other than the converting agent can be accommodated in the same container. For example, the sample treatment solution and the cleaving agent may be accommodated in the same container, and the dissolving solution and the converting agent may be accommodated in another container.

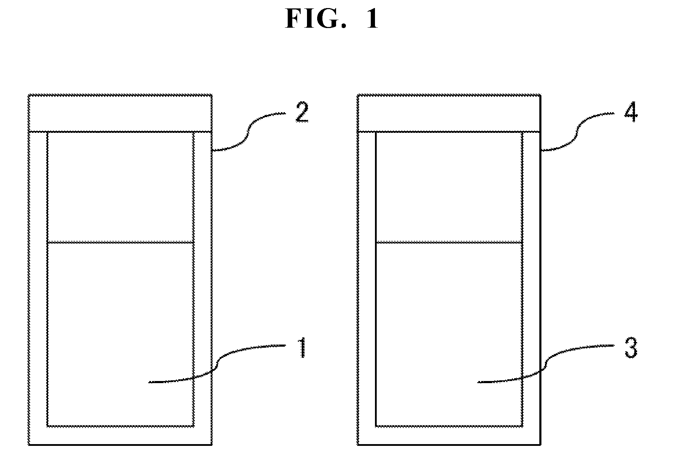

One example of the reagent kit is shown in Fig. 1. The reagent kit in Fig. 1 comprises a first reagent and a second reagent. The first reagent comprising a sample treatment solution 1 is accommodated in a reagent container 2. The second reagent comprising a converting agent 3 is accommodated in a reagent container 4. As described above, the reagent kit may further comprise a reagent container for accommodating the dissolving solution and the cleaving agent.

The method of detecting DNA methylation in a biological sample in this embodiment comprises a step (detecting step) of detecting methylation of a DNA contained in a sample for DNA methylation by using a polynucleotide hybridizing with a predetermined region of the DNA before the converting step, in addition to the above-mentioned method of preparing a sample for DNA methylation.

In the detecting step, methylation of a DNA is detected preferably by further using a polynucleotide hybridizing with a predetermined region of the DNA after non-methylated cytosine in the predetermined region of the DNA are converted into another base. The reliability of detection results can further be improved.

Another base converted from non-methylated cytosine by the converting agent, and 5-methylcytosine residues, are present in a DNA contained in a sample prepared by the sample preparation method described above. For distinguishing these converted another base from 5-methylcytosine residues, the amplification and/or detection of the DNA in the sample can be carried out to determine a methylated state of the predetermined region of the DNA.

Detection of DNA methylation is carried out by using a polynucleotide hybridizing specifically with a DNA sequence before conversion of non-methylated cytosine into another base (referred to hereinafter as non-converted sequence), and if necessary, a polynucleotide hybridizing specifically with a DNA sequence after conversion (referred to hereinafter as converted sequence). Methylation can be detected by nucleic acid amplification such as PCR and LAMP with said polynucleotide as a primer. Methylation may also be detected by using a DNA chip having said polynucleotide immobilized on a substrate.

Specific examples thereof include Methylation Specific PCR (MS-PCR) using a PCR primer which hybridizes specifically to DNA, which is methylated or non-methylated DNA, Bisulfite genome sequencing, COBRA (Combined Bisulfite Restriction Analysis), Ms-SNuPE (Methylation-Sensitive Single-Nucleotide Primer Extension), and the like. Alternatively, the methylation may be detected by the LAMP method (See, USP No. 6410278) using the above-mentioned polynucleotides.

When the MS-PCR is used, methylation is specifically detected as follows:
1) The sample for detection prepared from a biological sample is aliquoted into two tubes (the samples are designated as the samples for detection 1 and 2, respectively);
2) The polynucleotide which hybridizes specifically to a converted sequence, DNA polymerases, and dNTPs (dATP, dGTP, dTTP, and dCTP) are added to the sample for detection 1 to prepare a PCR reaction solution 1;
3) The polynucleotide which hybridizes specifically to a non-converted sequence, DNApolymerases, and dNTPs (dATP, dGTP, dTTP, and dCTP) are added to the sample for detection 2 to prepare a PCR reaction solution 2;
4) PCR is performed using the PCR reaction solutions 1 and 2; and
5) It is confirmed whether the sequence targeted is amplif iedby the below-mentioned detection method such as agarose gel electrophoresis.

As a result of the step defined by 5), it is confirmed that the region to which the polynucleotide hybridizes is not methylated in the case where the amplified DNA is observed in the PCR reaction solution 1, but not observed in the PCR reaction solution 2. On the contrary, it is confirmed that the region to which the polynucleotide hybridizes is methylated in the case where the amplified DNA is observed in the PCR reaction solution 2, but not observed in the PCR reaction solution 1.

The method of detecting the amplified DNA is not particularly limited, and it can be detected by publicly known methods. For example, an agarose gel electrophoresis, a real-time detection method withfluorescence-labeled probes (in this case, the above steps 4 and 5 can be performed at the same time), turbidimetry for measuring the turbid (turbidity) of by-product (magnesium pyrophosphate) in DNA synthesis, if necessary, a method of analyzing the pattern cleaved by enzymes, a method of determining a base sequence by direct sequence analysis, or the like can be used. When a specific band is difficult to distinguish due to presence of many nonspecific amplified bands, the specific band can be confirmed by Southern blot technique using probes in the region targeted for amplification.

Here, the above-mentioned polynucleotide can serve as a primer and it is also DNA or RNA of a linear single-stranded oligomer which hybridizes sequence-specifically to the complementary strand of DNA of modified or non-modified type (annealing). The polynucleotide has the suitable sequence and sufficient length so as to provide specific and efficient initiation of polymerization reaction (primer extension) during the amplification. The length of a primer is not particularly limited, however, it is preferably from 5 to 50 mer, more preferably from 10 to 30 mer when it is used for PCR. The primer is preferable to be a single strand, however, it is also possible to use a double strand when the strand is first separated. The primer can be prepared using any suitable methods, for example, a phosphotriester method and a phosphodiester method in routine use, or the automated embodiments which are generally known in the art.

The primers used are preferably designed so as to be substantially complementary to the predetermined region of a DNA and also to have a suitable content of GC. That is, the polynucleotide distinguishes particularly an untreated or unmodified DNA, a methylated DNA and a non-methylated DNA after the converting step. C will not be present in the sense primer, and G will not be present in the antisense primer, so a relatively small amount of C or G is usually contained in a sequence of the oligonucleotide primer used in the method of the present invention.

The case that bisulfite is used as a converting agent will be described hereinafter. Although non-methylatedcytosine are converted into uracils by bisulfite, 5-methylcytosine remains as it is without being converted. Therefore, the (non-methylated) genomic DNA having the converted uracil is used as a template to amplify nucleic acids with a polynucleotide which hybridizes the region containing uracil, resulting in amplified products. However, the nucleic acids are amplified with a polynucleotide which hybridizes the region containing 5-methylcytosine, resulting in no amplified products. On the other hand, the (methylated) genomic DNA containing 5-methylcytosine is not amplified with a polynucleotide which hybridizes the region containing uracil, resulting in no amplified products. However, the genomic DNA is amplified with a polynucleotide which hybridizes the region containing 5-methylcytosine, resulting in amplified products. Detection of the products by agarose gel electrophoresis can be performed to analyze whether the predetermined region of DNA are methylated or not.

As a predetermined region in DNA, the CpG island of a gene is preferable. This region is a DNA sequence relevant to gene transcription. The CpG island is mainly located in the upstream region of the exon of the gene (promoter region) and may be located in an intron or exon. The case where CpG island is methylated suggests that gene transcription is repressed. On the other hand, the case where CpG island is not methylated suggests that the gene is transcribed or is in a state that can be transcribed, because a transcription factor can be bound to a promoter region.

The gene described above is preferable to be at least one selected from the group consisting of an antioncogene, an oncogene and a gene encoding a tumor marker (hereinafter referred to as a tumor marker gene). Examples of an antioncogene include RB1 (Retinoblastoma 1 protein) gene, RRCA1 gene, APC (Adenomatous polyposis of the colon) gene, p53 gene, p16 gene, p15 gene, ERα (Estrogen Receptor 1) gene, E-cad (E-cadherin) gene, VHL (von Hippel-Lindau disease) gene, RASSF1A (RAS association domain family protein) gene, and the like. Examples of an oncogene include RAS gene, RAF gene, MYC gene, and the like. Examples of a tumor marker include CEA (Carcinoembryonic antigen-related cell adhesion molecule 5) gene, PSA (Prostate specific antigen) gene, CA15-3 gene, EpCAM (Epithelial cellular adhesion molecule) gene, and the like.

The method of detecting DNA methylation in this embodiment can be used to detect methylation of CpG islands in these genes, thereby being applied to genetic examination such as prediction of the risk for affection with diseases and drug susceptibility.

For example, when the CpG island of an antioncogene in a predetermined tumor cells is methylated and/or when the CpG island of an oncogene is not methylated, the tumor cells can be predicted to be malignant.

Further, when the CpG islands of the gene in which particular drugs such as anticancer agents are eliminated into the extracellular space and the gene in which drugs are degraded are methylated, resistance to the drugs in the cells is reduced; in other words, the cells can be determined to be sensitive to the drugs.

Detection of methylation of CpG island of tumor marker gene in predetermined tumor cells allows for identifying tumor types (identifying the primary focus).

Detection of methylation of CpG islands of the above-mentioned genes using a biological sample in which the presence or absence of cancer cells is not found allows for determining whether cancer cells are present or not, thereby detecting tumors at an early stage and determining risk of being affected by tumors.

In this way, for application to genetic examination such as prediction of the risk for affection with diseases and drug susceptibility, a biological sample having a high content of protein, such as serum and tissue colleted from a patient, is often used. Accordingly, when DNA methylation is detected by a detection sample prepared with only a conventional denaturant, it is highly possible that sufficient detection results cannot be obtained. When the rapidmethod is used for rapidly performing genetic examination, sufficient acquisition of detection results is made further difficult. Accordingly, the clinical application of the genetic examination using detection of DNA methylation has been difficult.

The method of detecting DNA methylation in this embodiment can achieve stable detection results in a short time by an easy operation. Accordingly, the detection method of the present invention can be suitably used in clinical examination such as genetic examination as described above. By using the rapid and easymethod of the present invention, research on DNAmethylation can be facilitated.

### EXAMPLES

### <Preparation of a biological sample>

A biological sample was prepared by adding 300 µl of human serum to 1 ng of a genomic DNA derived from cultured cell MDA231 derived from human breast cancer. As a control, a biological sample comprising 300 µl of human serum only without containing MDA231-derived genomic DNA was used.

### <Preparation of sample treatment solutions>

Sample treatment solutions A to F shown below were prepared. As a control, 320 µl of distilled water was used.
Sample treatment solution A:
   300 µl of an aqueous solution of urea at a final concentration of 4 M was mixed with 20 µl of 20 mg/ml aqueous proteinase K solution to obtain 320 µl of sample treatment solution A.
Sample treatment solution B:
   300 µl of an aqueous solution of guanidine hydrochloride at a final concentration of 4 M was mixed with 20 µl of 20 mg/ml aqueous proteinase K solution to obtain 320 µl of sample treatment solution B.
Sample treatment solution C:
   300 µl of an aqueous solution of SDS at a final concentration of 1% was mixed with 20 µl of 20 mg/ml aqueous proteinase K solution to obtain 320 µl of sample treatment solution C.
Sample treatment solution D:
   300 µl of an aqueous solution of SDS at a final concentration of 1% was mixed with 20 µl of distilled water to obtain 320 µl of sample treatment solution D.
Sample treatment solution E:
   300 µl of an aqueous solution of urea at a final concentration of 4M was mixed with 20 µl of distilled water to obtain 320 µl of sample treatment solution E.
Sample treatment solution F:
   300 µl of an aqueous solution of guanidine hydrochloride at a final concentration of 4M was mixed with 20 µl of distilled water to obtain 320 µl of sample treatment solution F.

### <Operating procedures of detection of DNA methylation>

Using the prepared sample treatment solutions A to F, the methylation of CpG islands in ERα gene and E-cad gene of cultured cell MDA231 derived from human breast cancer was detected in the following procedures.
1) The biological sample is mixed with any one of the sample treatment solutions A to F and stirred at 50°C for 1 hour.
2) 20 µl of 10 N NaOH aqueous solution is added thereto and incubated at 37°C for 10 minutes.
3) 600 µl of 10 M sodium bisulfite aqueous solution is added thereto and incubated at 80°C for 40 minutes.
4) The genome solution (sample for detection of DNA methylation) treated with the bisulfite is recovered with a DNA purification kit (trade name: QIAquik manufactured by Qiagen).
5) PCR is carried out with 2.0 µl of the recovered sample for DNA methylation. After the reaction, the reaction solution is stored at 4°C.
6) The reaction solution after PCR reaction is accommodated in a well on 2% agarose gel and electrophoresed.
7) The agarose gel after electrophoresis is stained with ethidium bromide, and a band of each of ERα gene and E-cad gene is confirmed.

### <Preparation of PCR reaction solution>

2. 0 µl of the sample for DNA methylation recovered in the operating procedure 4) above is mixed with the following materials to prepare 25 µl of a PCR reaction solution in PCR in the operating procedure 5) above.
10×PCR buffer (2.5 µl)
2.5 mM dNTP (2 µl)
10 µM sense primer (1.0 µl)
10 µM antisense primer (1.0 µl)
FastStart Taq DNA polymerase (0.2 µl, Roche 12032902001)
Water (16.3 µl)

As a primer for detection of methylation of CpG island in ERα gene, a non-converted sequence specific primer set (referred to hereinafter as methylation primer set) set forth in SEQ ID NOS: 1 and 2 below.

### <Methylation primer set>

Sense primer: TTTTGGGATTGTATTTGTTTTCGTC (SEQ ID NO: 1)

Antisense primer: AACAAAATACAAACCGTATCCCCG (SEQ ID NO: 2)

Using the PCR reaction solution prepared for detection of methylation of CpG island in ERα gene, PCR was carried out under the following conditions:
95°C, 9.5 minutes
45 cycles of the following steps i) to iii);

i) 95°C, 30 seconds
ii) 60°C, 15 seconds
iii) 72°C, 30 seconds

As a primer for detection of methylation of CpG island in E-cad gene, the following methylation primer set was used.

### <Methylation primer set>

Sense primer: 5'-TTAGGTTAGAGGGTTATCGCGT-3' (SEQ ID NO: 3)

Antisense primer: 5'-TAACTAAAAATTCACCTACCGAC-3' (SEQ ID NO: 4)

Using the PCR reaction solution prepared for detection of methylation of CpG island in E-cad gene, PCR was carried out under the following conditions:
95°C, 9.5 minutes
45 cycles of the following steps i) to iii);

i) 95°C, 30 seconds
ii) 60°C, 15 seconds
iii) 72°C, 30 seconds

### Example 1

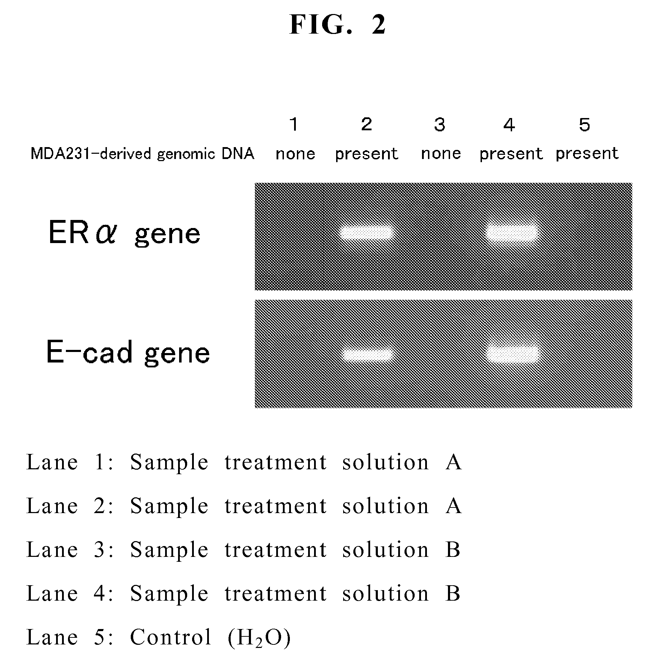

The detection results of methylated DNA with the sample treatment solution A, the sample treatment solution B or the control are shown in Fig. 2. As is evident from Fig. 2, the methylated DNA could be detected in both the ERα gene and E-cad gene with the sample treatment solutions A and B containing a protease proteinase K and a chaotropic ion urea or guanidine hydrochloride, but the methylated DNA could not be detected with distilled water as the control. The methylated DNA in the biological sample not containing the genomic DNA derived from MDA231 could not be detected with either the sample treatment solution A or B.

### Example 2

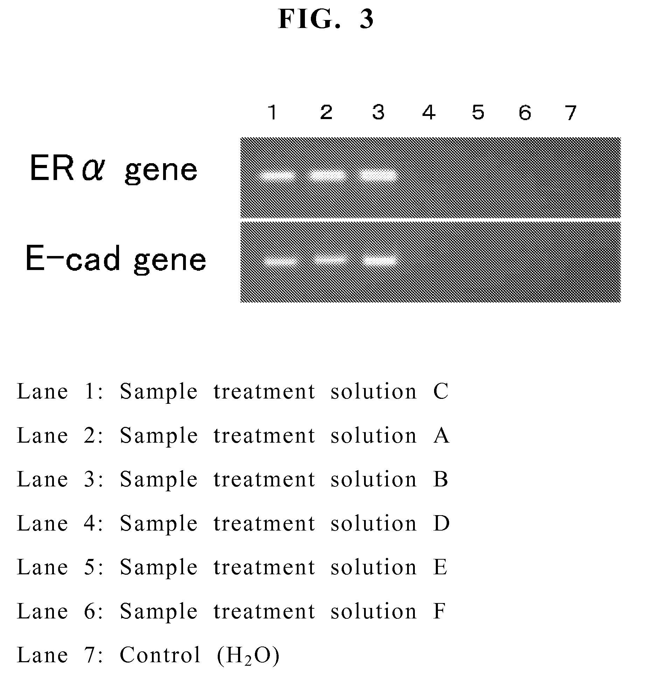

The detection results of methylated DNA in a biological sample containing the genomic DNA derived from MDA231 with each of the sample treatment solutions A to F or the control are shown in Fig. 3. As is evident from Fig. 3, the methylated DNA could be detected in both the ERα gene and E-cad gene with the sample treatment solutions A to C containing a protease proteinase K. However, the methylated DNA could not be detected with the proteinase K-free sample treatment solutions D to F, similar to the control.

From the above results, it was revealed that when a biological sample containing serum having a high concentration of protein is used, satisfactory detection results of DNA methylation cannot be obtained with a sample treatment solution containing only a surfactant or a chaotropic agent (SDS, urea and guanidine hydrochloride). It was revealed that by incorporating a protease into the sample treatment solution, stable detection results of DNA methylation can be obtained.

The foregoing detailed description and examples have been provided by way of explanation and illustration, and are not intended to limit the scope of the appended claims. Many variations in the presently preferred embodiments will be obvious to one of ordinary skill in the art, and remain within the scope of the appended claims and their equivalents.

## Claims

1. A sample treatment solution for preparing a sample for detection of DNAmethylation, comprising an aqueous solution including a protease.

2. The sample treatment solution according to claim 1, further comprising at least one selected from the group consisting of a surfactant and a chaotropic agent.

3. The sample treatment solution according to claim 2, wherein the surfactant is at least one selected from the group consisting of sodiumdodecyl sulfate, sodium tetradecyl sulfate, sodium dodecyl sulfonate, sodium tetradecyl sulfonate, sodium cholate, sodium deoxycholate, sodium taurocholate, and sodium taurodeoxycholate.

4. The sample treatment solution according to claim 2, wherein the chaotropic agent is at least one selected from the group consisting of guanidine thiocyanate, sodium thiocyanate, guanidine hydrochloride, sodium iodide, potassium iodide and urea.

5. The sample treatment solution according to any one of claims 1 to 4, further comprising a cleaving agent which is capable of cutting a DNA.

6. The sample treatment solution according to any one of claims 1 to 5, having a pH value of 7 to 10.

7. A reagent kit for preparing a sample for detection of DNA methylation, comprising:
a first reagent including the sample treatment solution of any one of claims 1 to 6; and
a second reagent including a converting agent for converting non-methylated cytosine contained in a DNA into another base.

8. The reagent kit according to claim 7, wherein the converting agent is a bisulfite.

9. The method according to claim 8, wherein the concentration of the bisulfite is 4 to 8 mM.

10. A method of preparing a sample for DNA methylation, comprising steps of:
mixing a sample treatment solution comprising an aqueous solution including a protease with a biological sample containing cells, and
converting non-methylated cytosine, which is contained in a DNA in the mixture obtained by the mixing step, into another base by adding a converting agent to the mixture.

11. The method according to claim 10, wherein the biological sample is serum, plasma, a biological tissue or a bodily secretion of mammary gland.

12. The method according to claim 10 or 11, wherein the cells are tumor cells.

13. The method according to any one of claims 10 to 12, further comprising a step of dissolving the biological sample with a dissolving solution including at least one selected from the group consisting of a surfactant and a chaotropic agent before the mixing step.

14. A method of detecting DNA methylation in a biological sample, comprising steps of:
mixing a sample treatment solution comprising a aqueous solution including a protease with a biological sample containing cells,
preparing a sample for detection of DNA methylation by adding a converting agent for converting non-methylated cytosine which is contained in a DNA in the mixture obtained by the mixing step, into another base to the mixture, and
detecting methylation of a DNA contained in the sample for DNA methylation by using a polynucleotide hybridizing with a predetermined DNA region before conversion into another base.

15. The method according to claim 13, wherein the predetermined DNA region is a region containing a CpG island of a gene.


SEQUENCE LISTING

<110>  SYSMEX CORPORATION

<120>  METHOD FOR PREPARING SAMPLE FOR DETECTING METHYLATION OF DNA

<130>  SYSM-018-EP

<140>  JP 2007-050748
<141>  2007-02-28

<160>  4

<170>  PatentIn version 3.1

<210>  1
<211>  25
<212>  DNA
<213>  Artificial

<220>
<223>  A sequence of a primer

<400>  1
ttttgggatt gtatttgttt tcgtc                                          25

<210>  2
<211>  24
<212>  DNA
<213>  Artificial

<220>
<223>  A sequence of a primer

<400>  2
aacaaaatac aaaccgtatc cccg                                           24

<210>  3
<211>  22
<212>  DNA
<213>  Artificial

<220>
<223>  A sequence of a primer

<400>  3
ttaggttaga gggttatcgc gt                                             22

<210>  4
<211>  23
<212>  DNA
<213>  Artificial

<220>

<223>  A sequence of a primer

<400>  4
taactaaaaa ttcacctacc gac                                              23